# EUROPEAN PATENT SPECIFICATION

(11) **EP 3 917 551 B1**
(45) Date of publication and mention of the grant of the patent: **22.04.2026**
(21) Application number: 20702795.4
(22) Date of filing: 31.01.2020
(51) Int. Cl.: A61K 35/747, A61P 29/00

(54) **LACTOCOCCUS LACTIS STRAINS FOR THE PREVENTION AND/OR THE TREATMENT OF VISCERAL PAIN**
LACTOCOCCUS-LACTIS-STÄMME ZUR VORBEUGUNG UND/ODER BEHANDLUNG VON VISZERALEN SCHMERZEN
SOUCHES LACTOCOCCUS LACTIS POUR LA PRÉVENTION ET/OU LE TRAITEMENT DE LA DOULEUR VISCÉRALE

(30) Priority: 01.02.2019 EP 19305125
(43) Date of publication of application: 08.12.2021
(73) Proprietor: Institut national de recherche pour l'agriculture, l'alimentation et l'environnement, 75007 Paris (FR); Institut National des Sciences Appliquées de Toulouse, 31077 Toulouse Cedex 4 (FR); CENTRE NATIONAL DE LA RECHERCHE SCIENTIFIQUE, 75016 Paris (FR); Ecole d'Ingénieurs de Purpan (EIP), 31076 Toulouse Cedex 3 (FR); Ecole Nationale Vétérinaire de Toulouse, 31300 Toulouse (FR); UNIVERSITE TOULOUSE III - PAUL SABATIER, 31062 Toulouse Cedex 9 (FR)
(72) Inventor: COCAIGN-BOUSQUET, Muriel, 31280 MONS (FR); EUTAMENE, Hélène, 31130 BALMA (FR); MERCIER BONIN, Muriel, 31400 TOULOUSE (FR); THEODOROU, Vassilia, 31120 PORTET-SUR-GARONNE (FR); LAROUTE, Valérie, 31000 TOULOUSE (FR); DAVERAN-MINGOT, Marie-Line, 31320 CASTANET (FR)
(74) Representative: Gevers & Orès
(86) International application number: PCT/EP2020/052466
(87) International publication number: WO 2020/157297

(56) References cited:
- YUNES R A ET AL: "GABA production and structure ofgadB/gadCgenes inLactobacillusandBifidobacteriumstrains from human microbiota", ANAEROBE, ELSEVIER, AMSTERDAM, NL, vol. 42, 26 October 2016 (2016-10-26), pages 197 - 204, XP029841286, ISSN: 1075-9964, DOI: 10.1016/J.ANAEROBE.2016.10.011
- VALÉRIE LAROUTE ET AL: "GABA Production in Lactococcus lactis Is Enhanced by Arginine and Co-addition of Malate", FRONTIERS IN MICROBIOLOGY, vol. 7, 6 July 2016 (2016-07-06), XP055600831, DOI: 10.3389/fmicb.2016.01050
- TESSÁLIA SARAIVA: "Lactococcus lactis NCDO2118 produces anti-hypertensive GABA and exerts acute hypotensive in spontaneously hypertensive rats", MEDICAL RESEARCH ARCHIVES, vol. 4, no. 7, 1 January 2016 (2016-01-01), XP055601497, ISSN: 2375-1916, DOI: 10.18103/mra.v4i7.620
- K. POKUSAEVA ET AL: "GABA-producing Bifidobacterium dentium modulates visceral sensitivity in the intestine", NEUROGASTROENTEROLOGY AND MOTILITY, vol. 29, no. 1, 25 July 2016 (2016-07-25), GB, pages e12904, XP055601020, ISSN: 1350-1925, DOI: 10.1111/nmo.12904
- TESSALIA DINIZ LUERCE ET AL: "Anti-inflammatory effects of Lactococcus lactis NCDO 2118 during the remission period of chemically induced colitis", GUT PATHOGENS, BIOMED CENTRAL LTD, LONDON, UK, vol. 6, no. 1, 29 July 2014 (2014-07-29), pages 33, XP021192311, ISSN: 1757-4749, DOI: 10.1186/1757-4749-6-33

## Description

The present invention relates to *Lactococcus lactis* strains which have the ability to reduce pain, in particular visceral pain; these strains are useful for the prevention and/or the treatment of visceral pain in an individual. The present invention also relates to new strains among these *Lactococcus lactis* strains and compositions containing them.

Visceral pain is pain associated with the viscera, which encompass the internal organs of the body. Here the term of viscera includes all the gastro-intestinal tract from the oral cavity to the anus. These organs also include, for example, the heart, lungs, reproductive organs, bladder, ureters, the organs of the digestive tract, preferably tongue, abdominal gut, liver, pancreas, spleen, and kidneys. Visceral pain is characterized by a vague, diffuse, dull, aching pain, which is localized but can have a tendency to radiate abdomen and/or back. It can be accompanied by a feeling of malaise, and, when severe, it induces strong autonomic phenomena such as sweating, vasomotor responses, bradycardia, nausea and vomiting. It is usually felt in the midline and deep in the body.

There are a variety of conditions in which abdominal or visceral pain may exist. For example, pancreatitis pain, labor pain, pain from abdominal surgery associated with ileus, pain in irritable bowel syndrome, abdominal pain in non-ulcer dyspepsia, or in dysmenorrhea. Likewise, liver pain, kidney pain, epigastric pain, pleural pain, and painful biliary colic, appendicitis pain may all be considered to be visceral pain. Substernal pain or pressure from early myocardial infarction is also visceral. Further, burning mouth syndrome (BMS) is a chronic idiopathic disorder characterized by burning sensation or dysesthesia in the tongue and other oral sites without clear etiology even if gastric origins are suspected. This BMS is accompanied by dry mouth, tingling, or dysgeusia. Considering the peripheral nervous alterations identified in BMS patients, some clinical trials have demonstrated the efficacy of topical GABA-A binding receptor ligands on BMS symptoms (de Castro *et al.,* 2014). Diseases of the stomach, duodenum or colon can cause visceral pain. Commonly encountered gastrointestinal (GI) disorders that cause visceral pain include functional bowel disorder (FBD) and inflammatory bowel disease (IBD). These GI disorders include a wide range of disease states that are currently only moderately controlled, including, with respect to FBD, gastro-esophageal reflux, dyspepsia, irritable bowel syndrome (IBS) and functional abdominal pain syndrome (FAPS), and, with respect to IBD, Crohn's disease, ileitis and ulcerative colitis, all of which regularly produce visceral pain.

According to Rome IV criteria, irritable bowel syndrome (IBS) is a functional gastrointestinal disorder with unknown etiology, affecting 10-20% of adults in most countries. IBS results in various symptoms that strongly affect the patient's quality of life (Dean *et al.,* 2005). It has been shown that stress is an important factor in the onset, maintenance and deterioration of IBS (Chang *et al.,* 2011), which results in significant morbidity and health care costs. IBS is a disorder of the brain-gut axis and clinical symptoms include abdominal pain and changes in bowel habits (*i.e.,* constipation, diarrhea) without structural abnormalities (Camilleri *et al.,* 2012). A subset of IBS patients, varying from 30% to 40%, were reported to exhibit enhanced sensitivity to colonic distension, which is noticeable through their reduced threshold for pain, increased intensity of sensations and/or exaggerated viscerosomatic referral in response to colonic distension (Mayer *et al.,* 1994, Mayer *et al.,* 2008, Bouin *et al.,* 2001, Zhou *et al.,* 2010). Thus, visceral hypersensitivity has been proposed as a key clinical marker accounting for the severity of bowel movements, bloating and abdominal pain symptoms experienced by IBS patients (Kuiken *et al.,* 2005, Posserud *et al.,* 2007, Camilleri *et al.,* 2008). For a better understanding of the IBS pathophysiology, acute and chronic stress animal models were developed mimicking IBS features, such as changes in visceral sensitivity and gut transit time (Gué *et al.,* 1997). In rat, acute restraint stress associated with visceral hypersensitivity to rectal distension is linked to central release of corticotrophin releasing factor (CRF) (Gué *et al.,* 1997). Current pharmacological treatments for these disorders (IBS and BMS) are to date mainly symptomdriven and their efficacy is generally low (Craig *et al.,* 2018).

It appears from the foregoing that there is a need for alternatives to pharmacological agents for the prevention and/or the treatment of visceral pain.

Some experimental evidence suggests that probiotics would be an effective treatment option to modulate abdominal pain in particular in IBS patients (Moayyedi *et al.,* 2010, Hungin *et al.,* 2013, Ford *et al.,* 2014, Didari *et al.* 2015); however, clinical results differ considerably among studies due to the strains of probiotics used, mixtures and dosages of these strains, as well as treatment duration and subtypes of IBS patients (Mazurak *et al.,* 2015).

Recently, the human gut commensal *Bifidobacterium dentium,* producing γ-aminobutyric acid (GABA), was shown to modulate sensory neuron activity in a rat fecal retention model of visceral hypersensitivity (Pokusaeva *et al.,* 2017). Moreover, the *Escherichia coli* strain Nissle 1917 was demonstrated to produce the C12AsnGABAOH lipopeptide, able to inhibit visceral sensitivity in mice (Pérez-Berezo *et al.,* 2017). However, no proof of the efficacy of the probiotic itself was given, only the analgesic properties of the C12AsnGABAOH lipopeptide was shown.

Thus, it remains useful to identify new therapeutic approaches to reduce visceral pain and that are easy to produce with a low production cost.

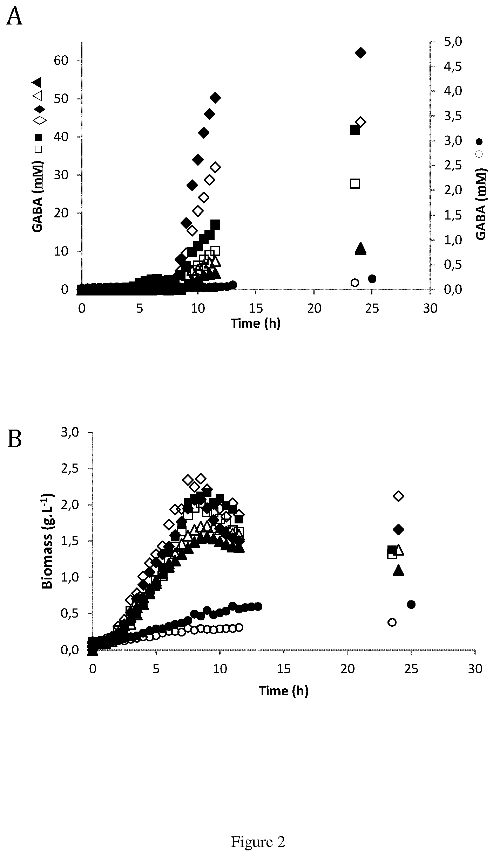

In this context, the Inventors focused on the study of the lactic acid bacteria, *Lactococcus lactis.* While the *Lactococcus lactis* subsp, *lactis* strains are known to produce GABA *in vitro* (Nomura *et al.,* 1999) and are theoretically, genetically able to express the pyridocal-5'-phosphate (PLP)-dependent enzyme glutamate decarboxylase (GAD), the enzyme which converts the glutamate in GABA, their effective capacity to express GAD and produce GABA is very aleatory for reasons that are not known. Indeed, the Inventors showed that despite the strong similarity of the *gad* operon involved in GABA biosynthesis between *L. lactis* NCDO2727 and NCDO2118 strains, GABA production under similar culture conditions was very different with a very weak amount of GABA produced by the NCDO2727 strain (GABA concentration always below 0.2 mM in Figure 2A). In a same way, a high GAD activity was obtained in the NCDO2118 strain, while in the NCDO2727 strain, the amount was extremely low (Table 3).

Frequently encountered in dairy products, *L. lactis* is one of the most ingested bacteria (Mills *et al.,* 2010, Laroute *et al.,* 2017). This bacterium is commonly found in dairy products such as cheese or dairy fermented products, including fermented milk.

Although not considered as a commensal bacterium, *L. lactis* was found to persist transiently in the gut, depending on the strain under study (Wang *et al.,* 2011, Radziwill-Bienkowska *et al.,* 2016, Zhang *et al.,* 2016).

The Inventors identified new *L. lactis* strains able to produce high amounts of GABA, *i.e.,* at least 10 mM of γ-aminobutyric acid (GABA) in a time of culture lower or equal of 24 hours, and they have shown that these strains possess the surprising and unexpected ability to reduce visceral pain. Thus, these *L. lactis* strains represent new agents for preventing and/or treating visceral pain.

Moreover, the Inventors have demonstrated that a *L. lactis* stain, NCDO2727, which produces low amount of GABA, do not possess such advantageous properties.

Accordingly, an object of the present invention are two Lactococcus lactis strains able to produce at least 10 mM of γ-aminobutyric acid (GABA) in a time of culture lower or equal to 24 hours, for use in the prevention and/or the treatment of visceral pain, in an individual.

Preferably, the *Lactococcus lactis* strain of the present invention is able to produce at least 10 mM of GABA, more preferably 20 mM of GABA, still more preferably 40 mM of GABA and most preferably at least 60 mM of GABA in a time of culture lower or equal to 24 hours (Figure 2A).

The ability to produce GABA may be assessed by culturing a *Lactococcus lactis* strain in a medium M17 supplemented with glutamate, arginine, glucose and NaCl in a fermenter. In the first hours of culture, the pH is maintained at 6.6, GAD is induced and accumulated. At 7-8 hours of culture, the strains are collected for individual treatment then pH is decreased at 4.6 to quantify GABA production rate. The concentration of GABA is measured over time until 24 hours.

The preparation of culture media and the definition of the culture conditions allowing the growth and the induction of GAD expression in the strains of *Lactococcus lactis* of the present invention are well known by the person skilled in the art; such culture medium may be adapted to each specific *L. lactis* strain; for example, for the strain CNCM I-5388, the addition of arginine and NaCl to a basic M17 culture medium has been proven to induce the expression of GAD; other strains such as CNCM I-5386 are able to enhance the GAD expression at a level similar to NCDO2118 when cultured on this M17 culture medium without addition of any specific compound.

An individual according to the invention is a mammal or a bird; for example, the individual may be an animal from a livestock breeding, such as poultry, or a pet; according to a specific embodiment, the individual is a human.

The two specific L. lactis strains of the present invention are characterized in that the activity of the pyridocal-5'-phosphate (PLP)-dependent enzyme glutamate decarboxylase (GAD) produced by said strain is at least 20 µmole/min.mg of protein in a time of culture comprised between 7 hours and 8 hours.

Preferably, the activity of the GAD produced by *Lactococcus lactis* strain in the present invention is at least 20 µmole/min.mg of protein, more preferably 40 µmole/min.mg, still more preferably 60 µmole/min.mg and in an increasing order at least 100, at least 200, at least 300, at least 400, at least 500, at least 600, at least 700 µmole/min.mg and most preferably at least 820 µmole/min.mg in a time of culture comprised between 7 hours and 8 hours.

Preferably, the strain *L. lactis* of the present invention is characterized in that the GABA production rate at pH 4.6 by said strain is at least of 1 mmole/h/g of cell dry weight in a time of culture comprised between 8.5 hours and 11.5 hours.

Preferably, the specific production rate of GABA in the present invention is at least 1 mmole/h/g of cell dry weight, more preferably 3 mmole/h/g of cell dry weight and most preferably at least 7 mmole/h/g of cell dry weight in a time of culture comprised between 8.5 hours and 11.5 hours.

Methods for assessing the GAD activity are known in the art as shown in Example 1. The GABA production rate is a specific rate calculated between 8.5 hours and 11.5 hours (mean value).

The strain *L. lactis* in the present description is selected in the group consisting of: CNCM I-5388, CNCM I-5386 and NCDO2118, the claimed invention being limited to CNCM I-5388 and CNCM I-5386, and more preferably said *L. lactis* strain is the strain CNCM I-5388.

The strains CNCM I-5388 and CNCM I-5386 were deposited according to the Budapest Treaty, at CNCM (Collection Nationale de Cultures de Microorganismes, 25 rue du Docteur Roux, Paris) on November 29, 2018 for CNCM I-5386 and on December 13, 2018 for CNCM I-5388.

The *L. lactis* strains of the present invention are of a particular interest in preventing or treating visceral pain.

The prophylactic and therapeutic effect of the *L. lactis* strains of the invention is obtained in presence of free glutamate; for example, the amount of free glutamate is about 4 mg per 10⁹ cfu of *L lactis* and per day or is about 17 mg/kg bw/day.

As demonstrated in Example 2, free glutamate *per se* does not produce any beneficial or unwanted effect on basal and stress-induced colorectal activity.

Depending of the continent and diet culture, classic diet provides different levels of free glutamate (average population intakes of free glutamate range from 5.5 mg/kg bw/day (elderly Austrians) to 37 mg/kg bw/day (toddlers in Belgium); high-level intakes range up to 82 mg/kg bw/day for other children in Belgium, 77 mg/kg bw/day for Danish infants, and 56 mg/kg bw/day for other children in Bulgaria; moreover, from natural free glutamate and dietary protein, a range of 81 to 155 mg/kg bw/day can be ingested in adults. David R. Tennant, Ann. Nutr. Metab. 2018;73:21-28). Therefore, according to these data, the Inventors considered that *L. lactis* strains of the invention can be administered alone, i.e. without the addition of free glutamate to exert an efficient prevention or a reduction of abdominal or visceral pain.

Alternatively, in case of free glutamate-free diet or diet especially poor in free glutamate, the *L. lactis* strains of the invention may be administered together with free glutamate. Accordingly, in a specific embodiment, another object of the present invention is the combination of strain *L. lactis* of the present invention and of between 10 and 20 mg/kg bw/day, in particular 17 mg/kg bw/day of free glutamate for use in the prevention and/or the treatment of visceral pain, in an individual.

For the different aspects of present invention, visceral pain may be caused by an injury, an infection, an inflammation, a disease or by a thermal stimulus, a mechanical stimulus, an electric stimulus a chemical stimulus or a radioactive stimulus.

Visceral pain in the invention is in particular those observed in chronic abdominal and pelvic pain disorders, including irritable bowel syndrome (IBS) but also burning mouth syndrome (BMS). IBS patients exhibit significantly lower response thresholds to provocative stimuli (*i.e.,* rectal distension), complain of increased sensitivity during normal organ function, and present increased tenderness in expanded areas of somatic (abdominal) referral (*i.e.,* both visceral and somatic hypersensitivity). Functional visceral (gastrointestinal) disorders resulted from altered central nervous system processing and/or dysregulated central modulation. In this cascade of events, visceral inputs of primary afferent nociceptors located in the Dorsal Root Ganglia (DRG) are responsible for changes in the perception of pain. However, all chronic diseases such as hepatitis, peptic ulcer, IBD (Crohn's disease and ulcerative colitis), IBS in which abdominal and/or back pain is a principal complaint and all the perceived sensations are initiated by activity in peripheral sensory (afferent) neurons (Keszthelyi D. *et al.,* 2012). Regarding Burning mouth syndrome (BMS) that is an important chronic pain disorder that affects more than 1 million people in the United States (Miziara *et al.,* 2009). BMS is characterized by both positive (burning pain, dysgeusia and dysesthesia) and negative (loss of taste and paraesthesia) sensory symptoms involving the lips and tongue, mainly the tip and anterior two-thirds. BMS may be classified into three types (Sun *et al.,* 2013). In Type 1, patients are free of pain on waking but experience increasing symptoms as the day goes on. In Type 2, patients have continuous pain throughout the day; this type accounts for 55% of patients and has the strongest association with psychological disorders (Coculescu *et al.,* 2014). In Type 3, patients have intermittent symptoms with pain-free periods during the day. Moreover, these BMS patients frequently have depression, anxiety, sometimes diabetes, and even nutritional/mineral deficiencies.

According to an embodiment of the invention, visceral pain is caused by a gastrointestinal disorder, such as an Irritable Bowel Syndrome or Burning Mouth Syndrome characterized by burning of the oral mucosa in the absence of underlying dental or medical causes. This is also the case with a functional and organic dyspepsia, narcotic bowel syndrome, IBD, colonic cancer, chronic pancreatitis, diverticulitis, appendicitis, ovarian cyst.

It is known that visceral pain is increased in various psychological stress conditions (acute and chronic).

An acute partial restraint stress increases visceral sensitivity (hypersensitivity) and also reduces the threshold of the sensitivity (allodynia).

According to an embodiment of the invention, the *L. lactis* strains in the present invention are useful when visceral pain is linked to psychological stress events and/or anxiety. The model of the psychological stress events and/or anxiety is an acute partial restraint stress (PRS).

PRS is considered as a mild non-ulcerogenic model (Williams *et al.,* 1987) and consists of under light anesthesia with ethyl ether, constraining animal body movements. For this, the upper forelimbs of the animal are taped up to the thoracic trunk in order to constrain animal body movements during 2 hours. Rats are then replaced in their home cages. In rats PRS is reflected by both an increase of adrenocorticotropic hormone (ACTH) and corticosterone plasmatic concentrations (Sun *et al.,* 2006), associated with visceral hypersensitivity to rectal distension linked to central release of corticotrophin releasing factor (CRF), (Gue *et al.,* 1997), as well as increase in gut permeability (Ait-Belgnaoui *et al.,* 2005).

Indeed, the Inventors showed that *L. lactis* NCDO2118, producing high level of GABA, prevents visceral hypersensitivity in an acute-stress rat model, whereas *L. lactis* NCDO2727, producing low level of GABA, is not able to induce such an effect.

Further, the NCDO2118 *ΔgadB* mutant strain, unable to produce GABA, failed to prevent visceral hypersensitivity in the same model.

In one aspect of the invention, the *L. lactis* strains of the present invention are characterized in that the effect produced by these strains is obtained from 10 days, more preferably from 7 days, still more preferably from 5 days and most preferably from 4 days of the treatment of visceral pain linked to psychological stress events and/or anxiety.

Indeed, the Inventors showed that the beneficial effect produced by CNCM I-5388 is obtained from 5 days of the treatment of visceral pain linked to an acute stress, and the effect produced by NCDO2118 is obtained from 10 days of such treatment.

Another aspect of the invention is the *Lactococcus lactis* strain as defined above, for use in the prevention and/or the treatment of visceral pain due to burning mouth syndrome (BMS).

An object of the present invention is also a cell fraction obtained from the *Lactococcus lactis* strain as defined above, for use in the prevention and/or the treatment of visceral pain and in particular for use in the prevention and/or the treatment of visceral pain linked to psychological stress events and/or anxiety.

Said cell fraction is in particular preparations containing bacterial enzymes, especially GAD, obtained from cultures of *L. lactis* of the invention. It may also be culture supernatants or fractions of these strains. The cell fractions can be prepared according to the methods known for the person skilled in the art. In a non-limited way, these methods generally include a step of lysis of the bacteria obtained after culture and a step of separation of the fractions containing the membranes of the said bacteria from the total lysate obtained after the lysis step, in particular by centrifugation or filtration.

Another aspect of the invention is the cell fraction obtained from *Lactococcus lactis* strain as defined above, for use in the prevention and/or the treatment of visceral pain due to burning mouth syndrome (BMS).

An object of the present invention is also a composition comprising the *Lactococcus lactis* strain according to the present invention or the cell fraction according to the present invention and optionally free glutamate preferably in an amount of between 10 and 20 mg/kg bw/day, in particular of 17 mg/kg bw/day, for use in the prevention and/or the treatment of visceral pain.

In the composition of the invention, said strain can be used in the form of whole bacteria which may be living or dead. Alternatively, said strain can be used in the form of a bacterial lysate. Preferably the bacterial cells are present as living, viable cells.

The composition of the invention can be in any form suitable for administration, in particular oral administration. This includes for instance solids, semi-solids, liquids, and powders.

When the bacteria are in the form of living bacteria, the composition given per day may comprise a content of the *L. lactis* strain according to the present invention allowing the administration of at least 10⁶ colony forming units (cfu) of said *L. lactis* strain, preferably at least 10⁷ cfu, still more preferably at least 10⁸ cfu, and most preferably at least 10⁹ cfu.

Another aspect of the invention is the composition comprising the *Lactococcus lactis* strain according to the present invention or the cell fraction according to the present invention, for use in the prevention and/or the treatment of visceral pain due to burning mouth syndrome (BMS).

Also disclosed is a method for preventing and/or treating visceral pain, comprising administering to a mammal in need thereof, a therapeutic amount of the *Lactococcus lactis* strain of the invention or the cell fraction of the invention, as defined above. Determination of a therapeutic amount is well known from the person skilled in the art, especially in view of the detailed disclosure provided herein.

An object of the present invention is also the use of the *Lactococcus lactis* strain of the invention or the cell fraction of the invention, as defined above for the manufacture of a medicament for preventing and/or treating visceral pain.

As used herein, the treatment or prevention encompasses inter alia: preventing and/or decreasing visceral pain.

The composition of the present invention can be a pharmaceutical composition or a nutritional composition, including food products such as food supplements and functional food. The food products comprise dairy products and dairy fermented products.

A pharmaceutical composition can be in an oral form such as tablets, capsules, powders, syrups effervescent compositions and sprays. Said pharmaceutical composition can also be in sublingual and buccal forms such as sublingual tablets and solution compositions that are administered under the tongue and buccal tablets that are placed between the cheek and gum.

A food supplement designates a product made from compounds usually used in foodstuffs, but which is in the form of tablets, powder, capsules, potion or any other form usually not associated with aliments, and which has beneficial effects for one's health.

A functional food is an aliment which also has beneficial effects for one's health. In particular, food supplements and functional food can have a physiological effect, protective or curative, against a disease or a pain.

The composition can be in the form of a liquid or present in the form of a dry powder obtained by drying the liquid.

In a preferred embodiment, the product is a fresh product. A fresh product, which has not undergone severe heat treatment steps, has the advantage that the bacterial strains present are in the living form.

The Inventors have also identified two new *Lactococcus lactis* strains having the specific abilities discussed previously.

Thus, the present invention also provides a *Lactococcus lactis* strain, characterized in that said strain is selected in the group consisting of: CNCM I-5388, CNCM I-5386.

The present invention also encompasses a cell fraction obtained from the *Lactococcus lactis* strain selected in the group consisting of: CNCM I-5388, CNCM I-5386.

The present invention also provides a composition comprising said *Lactococcus lactis* strain as defined above or said cell fraction as defined above.

Said composition can comprise at least 10⁶ colony forming units (cfu), preferably at least 10⁷ cfu, more preferably at least 10⁸ cfu, and most preferably at least 10⁹ cfu per gram dry weight of the composition.

The composition as defined above can be a pharmaceutical composition or a nutritional composition, including food products as defined above.

Said composition can be in the form of a liquid or present in the form of a dry powder obtained by drying the liquid.

In a preferred embodiment, the product is a fresh product.

An object of this invention is related to a pharmaceutical composition comprising the *Lactococcus lactis* strain or the cell fraction as defined above and a pharmaceutically acceptable vehicle; optionally said composition also comprises free glutamate.

In an embodiment of the invention, the composition as defined above is for use as a medicament. The formulation of said medicament is adapted allowing local treatment in the form of a spray or tablets to melt under the tongue.

Said composition is particularly useful for preventing and/or treating visceral pain.

Also disclosed in a method of treatment for preventing and/or treating visceral pain as defined above, comprising the administration of the *Lactococcus lactis* strain or the cell fraction or the composition as defined above to an individual.

The present invention also relates to the *Lactococcus lactis* strain or the cell fraction of the invention, optionally in association with free glutamate, for the preparation of a medicine for the prevention and/or the treatment of visceral pain as defined above.

The present invention will be understood more clearly from the further description which follows, which refers to examples illustrating the capacity of the *L. lactis* strains in the present invention for reducing visceral pain, as well as to the appended figures.

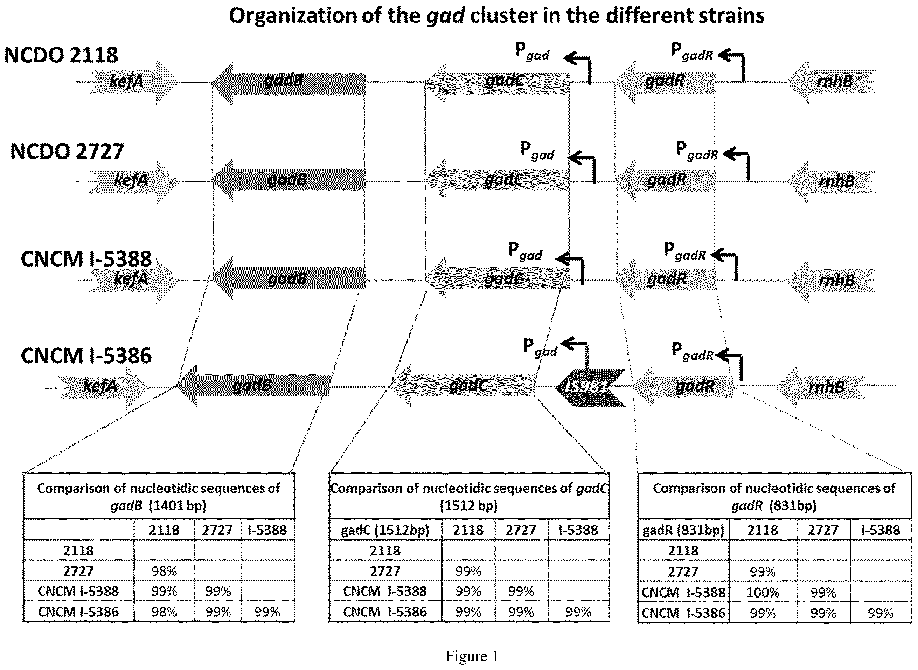

**Figure 1** shows the organization of the *gad* cluster in the different strains.
**Figure 2** shows the GABA production (mM) (A) and evolution of biomass (g/L) (B) during growth of *L. lactis* NCDO2118 (square), CNCM I-5388 (diamond), CNCM I-5386 (triangle) or NCDO2727 (circle) in M17 supplemented with free glutamate (8 g/L), arginine (5 g/L), glucose (45 g/L) and NaCl (300 mM). Independent duplicates (filled symbols: first experiment and open symbols: second experiment).

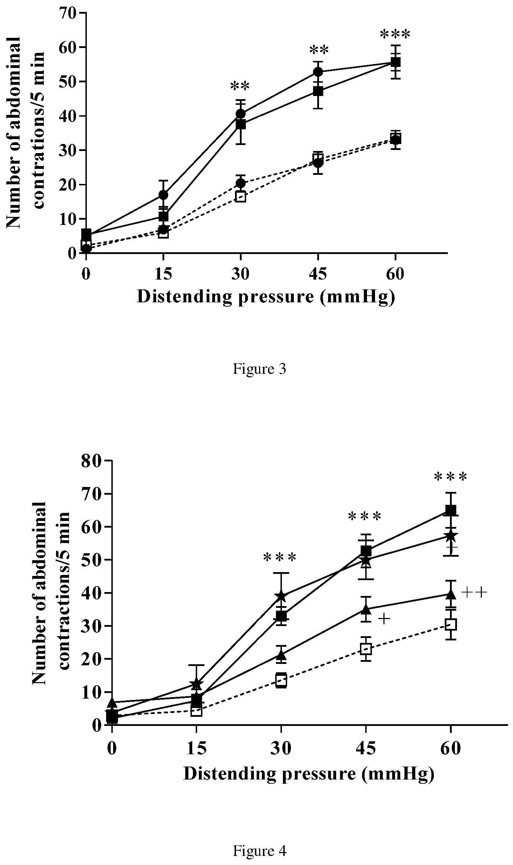

**Figure 3** shows the effect of 10-day oral administration of free glutamate (2 mg in the diet and 2 mg co-administrated with *L lactis*) on and PRS-induced colorectal sensitivity of vehicle-treated rats at all the distension pressures of CRD (from 15 to 60 mmHg). Data are expressed as means ± SEM (n=10 for the "vehicle" group (open square, dashed line); n=10 for the "PRS+vehicle" group (black square, solid line); n=11 for the "vehicle+free glutamate" group (black circle, dashed line); n=11 for the "PRS+vehicle+free glutamate" group (black circle, solid line)). **P<*0.05; ***P<*0.01; ****P<*0.001 *vs.* basal values for animals treated with vehicle.
**Figure 4** shows the effect of 10-day oral administration of *L. lactis* NCDO2118 and *L. lactis* NCDO2727 on PRS-induced visceral hypersensitivity at all the distension pressures of CRD (from 15 to 60 mmHg). Data are expressed as means ± SEM (n=9 for the "vehicle" group (white square, dashed line) and "PRS+vehicle" group (black square, solid line); n=12 for the "PRS+NCDO2118+free glutamate" group (black triangle, solid line); n=8 for the "PRS+NCDO2727+free glutamate" group (black star, solid line)). ***P<0.001 *vs.* basal values for animals treated with vehicle. *⁺P<*0.05; *⁺⁺P<*0.01 *vs.* values for stressed animals treated with vehicle.

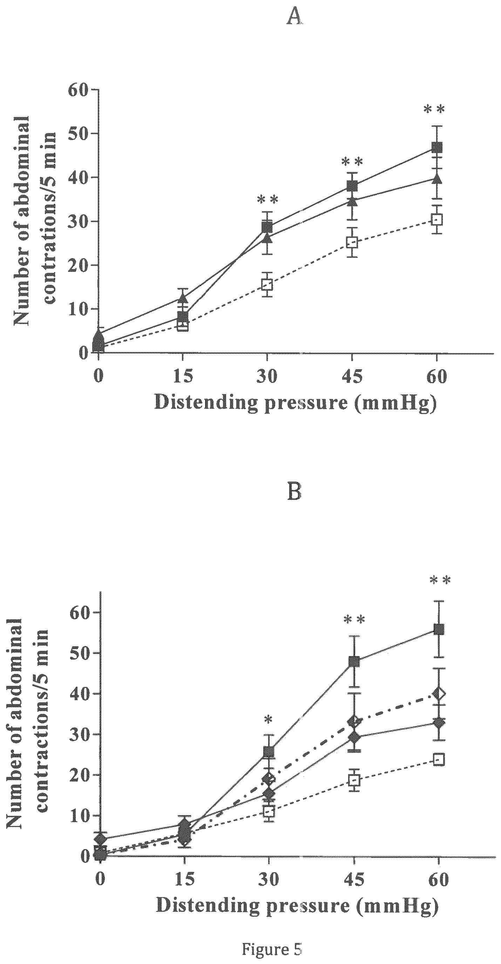

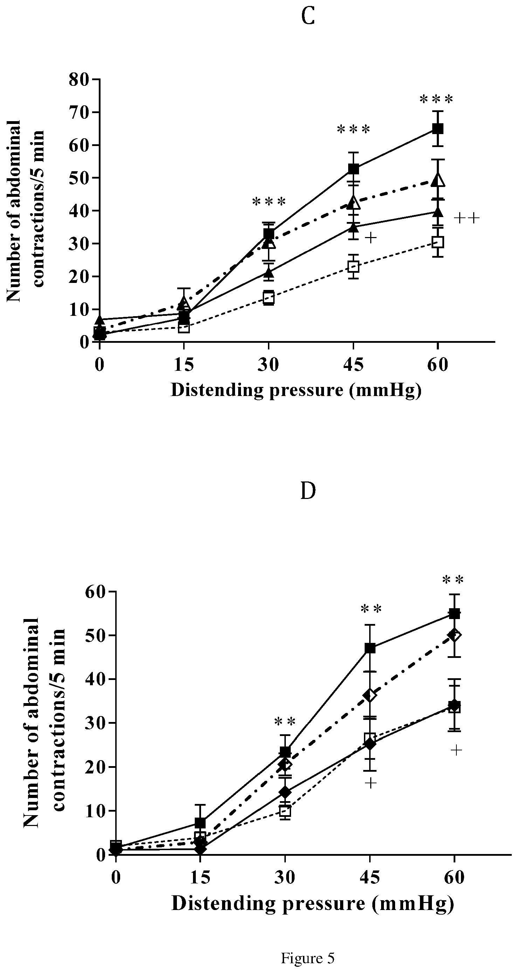

**Figure 5** shows the effect of 5-day or 10-day-oral administration of *L. lactis* NCDO2118 and *L. lactis* CNCM I-5388 on PRS-induced visceral hypersensitivity at all the distension pressures of CRD (from 15 to 60 mmHg). **A.** Results obtained with *L. lactis* NCDO2118 at 5 days. Data are expressed as means ± SEM (n=11 for the "vehicle" group (white square, dashed line), n=11 for the "PRS+vehicle" group (black square, solid line) and n=11 for the "PRS+NCDO2118+free glutamate" group (black triangle, solid line)). ***P*<0.01 significantly different *vs*. basal values for animals treated with vehicle. **B.** Results obtained with *L. lactis* CNCM I-5388 at 5 days. Data are expressed as means ± SEM (n=7 for the "vehicle" group (white square, dashed line); n=7 for the "PRS+vehicle" group (black square, solid line); n=7 for the "PRS+CNCM I-5388" group (black/white diamond, dash-dotted line) and "PRS+CNCM I-5388+free glutamate" (black diamond, solid line)). **P<*0.05; ***P<*0.01 *vs.* basal values for animals treated with vehicle, **C.** Results obtained with *L. lactis* NCDO2118 at 10 days. Data are expressed as means ± SEM (n=9 for the "vehicle" group (white square, solid line); n=9 for the "PRS+vehicle" group (black square, solid line); n=12 for the "PRS+NCDO2118+free glutamate" group (black triangle, solid line); n=8 for the "PRS+NCDO2118" group (black/white triangle, dash-dotted line))****P*<0.001 *vs.* basal values for animals treated with vehicle. *⁺P<*0.05; *⁺⁺P<*0.01 *vs.* values for stressed animals treated with vehicle. **D.** Results obtained with *L. lactis* CNCM I-5388 at 10 days. Data are expressed as means ± SEM (n=7 for the "vehicle" group (white square, dashed line); n=7 for the "PRS+vehicle" group (black square, solid line); n=8 for the "PRS+CNCM I-5388" group (black/white diamond, dash-dotted line) and n=7 for the "PRS+CNCM I-5388+free glutamate" (black diamond, solid line).***P*<0.01 *vs.* basal values for animals treated with vehicle. *⁺P<*0.05 *vs.* values for stressed animals treated with vehicle.

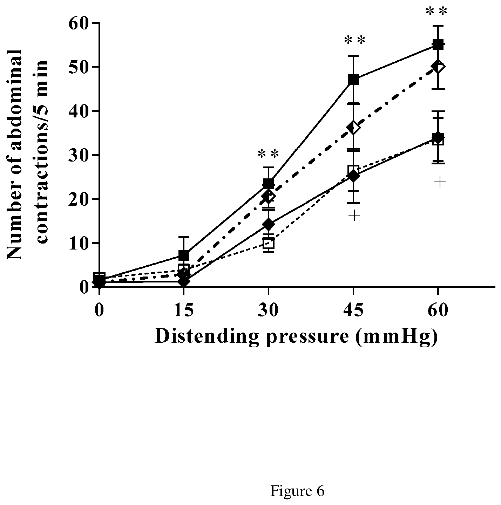

**Figure 6** shows the effect of GABA-producing *L. lactis* NCDO2118 and the non-GABA producing NCDO2118 *ΔgadB* mutant (10⁹ cfu per day) on PRS-induced visceral hypersensitivity at all the distension pressures of CRD (from 15 to 60 mmHg). Data are expressed as means ± SEM (n=13 for the "vehicle" group (white square, dashed line) and "PRS+vehicle" group (black square, solid line); n=14 for the "PRS+NCDO2118+free glutamate" group (black triangle, solid line); n=13 for the "PRS+ NCDO2118 *ΔgadB*+free glutamate" group (inverted triangle, dash-dotted line)). ***P<*0.01; *****P<*0.0001 *vs.* basal values for animals treated with vehicle. ⁺⁺*P<*0.01; ⁺⁺⁺*P<*0.001 *vs.* values for stressed animals treated with vehicle ^{$}*P<*0.05 *vs.* values for stressed animals treated with NCDO2118.

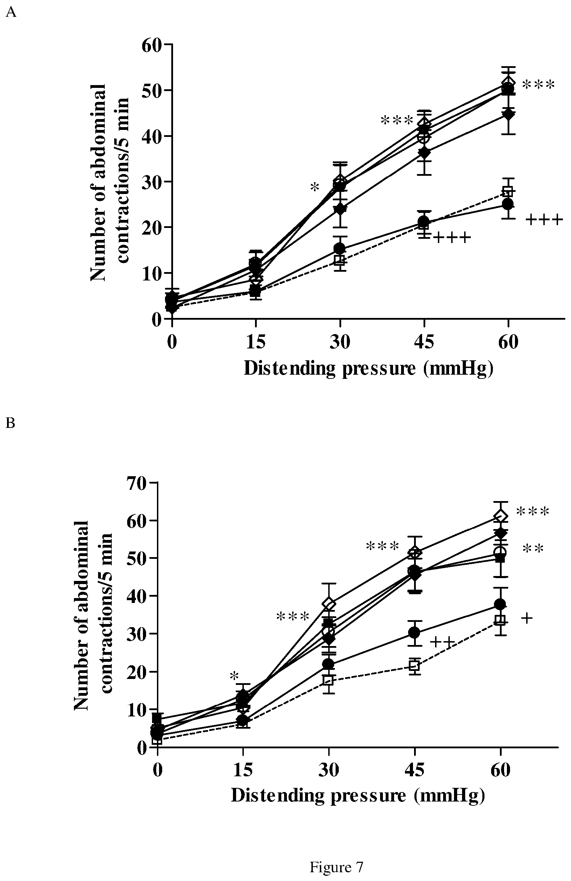

**Figure 7** shows the effect of 5-day or 10-day-oral administration of *L. lactis* CNCM I-5388 on PRS-induced visceral hypersensitivity at all the distension pressures of CRD (from 15 to 60 mmHg). **A.** Results obtained with *L. lactis* CNCM I-5388 at 5 days. Data are expressed as means ± SEM (n=10 for the "vehicle" group (white square, dashed line), n=10 for the "PRS+vehicle" group (black square, solid line), n=10 for the "PRS+CNCM I-5388" group (white ring, solid line), n=9 for the "PRS+CNCM I-5388+free glutamate" group (black ring, solid line), n=9 for the "PRS+CNCM I-5388+free glutamate+inhibitor" group (white rhombus, solid line), n=9 for the "PRS+CNCM I-5388 *Δgad* mutant+free glutamate" group (black rhombus, solid line). *P<0.05; ***P<*0.01; *****P<*0.0001 *vs.* basal values for animals treated with vehicle. ⁺⁺⁺*P<*0.001 *vs*. values for stressed animals treated with vehicle. **B.** Results obtained with *L. lactis* CNCM I-5388 at 10 days. Data are expressed as means ± SEM (n=10 for the "vehicle" group ((white square, dashed line), n=10 for the "PRS+vehicle" group (black square, solid line), n=9 for the "PRS+CNCM I-5388" group (white ring, solid line), n=9 for the "PRS+CNCM I-5388+free glutamate" group (black ring, solid line), n=9 for the "PRS+CNCM I-5388+free glutamate+inhibitor" group (white rhombus, solid line), n=9 for the "PRS+CNCM I-5388 *Δgad* mutant+free glutamate" group (black rhombus, solid line)). *P<0.05; ***P<*0.01; *****P<*0.0001 *vs.* basal values for animals treated with vehicle. *⁺P<*0.05; *⁺⁺P<*0.01 *vs.* values for stressed animals treated with vehicle.

### EXAMPLE 1: Physiological characterization of bacterial suspensions of interest

### 1. Materials and Methods:

### 1.1.Bacterial strains, medium and culture conditions:

The data set is composed of four strains belonging to the *Lactococcus lactis* subsp. *lactis.* The two strains, NCDO2118 and NCDO2727 were isolated from vegetables while CNCM I-5388 and CNCM I-5386 have a dairy origin (raw milk and whey respectively).

In these strains, the *gad* cluster involved in GABA biosynthesis is located on the chromosome between *kefA* and *rnhB* genes (Figure 1). The *gad* cluster is composed of *gadR, gadC* and *gadB* encoding respectively the operon regulator, glutamate/GABA antiporter and glutamate decarboxylase. The same cluster organization is retained in these strains except for CNCM I-5386. Indeed, a copy of the insertion sequence IS981 has been found in the promoter of the *gadCB* operon. This insertion sequence contains outward -35 promoter component and its insertion at the correct distance from the native -10 box can modify the strength of the promoter and its regulation. The nucleotide sequences of *gadR, gadC* and *gadB* genes are highly similar in the 4 strains with more than 98% identity (Figure 1).

Bacterial cultures were performed in duplicate in 2-L Biostat B-plus bioreactor (Sartorius, Melsungen, Germany) in medium M17 (Table 1) supplemented with 55 mM (8 g/L) free glutamate, 29 mM (5 g/L) arginine, 250 mM (45 g/L) glucose and 300 mM NaCl. Cultures were incubated at 30°C.

**Table 1: Composition of medium M17.**

| Ingredients | g/L |
|---|---|
| Tryptone | 2.5 |
| Peptic digest of animal tissue | 2.5 |
| Papaic digest of soybean meal | 5 |
| Meat extract | 5 |
| Yeast extract | 2.5 |
| Ascorbic acid | 0.5 |
| Magnesium sulphate | 0.5 |
| Sodium glycerophosphate | 19 |

Fermentations were carried out under oxygen-limiting conditions, with air in the gaseous phase but without air bubbling. pH was maintained at 6.6 by KOH addition for 8 h, then pH was modified and regulated at 4.6.

Culture was inoculated with cells from pre-cultures grown in Erlenmeyer flask on similar medium, harvested during the exponential phase and concentrated in order to obtain an initial optical density (OD) at 580 nm of 0.25 in the fermenter.

Bacterial growth was estimated by spectrophotometric measurements at 580 nm (Libra S11, Biochom, 1 Unity of absorbance is equivalent to 0.3 g/L).

Samples were collected every 30 min for HPLC measurement of GABA concentration in the growth medium.

Cellular samples for oral administration were prepared as follows. Cells were harvested before the pH modification (*i.e.* at 7 h for NCDO2118 and NCDO2727).

The culture volume required for approximately 3x10¹¹ cells was centrifuged at 5000 g and 4°C for 10 min to pellet the bacterial cells.

Cells were washed twice with 0.9% NaCl and suspended in 0.9% NaCl containing 15% glycerol to a final concentration of 10x10⁹ cfu/mL and stored at -20°C until use.

### 1.2.GABA extraction and quantification:

GABA concentration in culture supernatant or reaction mixture of enzyme assay was measured by HPLC (Agilent Technologies 1200 Series, Waldbronn, Germany).

Prior to HPLC, proteins were precipitated by adding four volumes of methanol to one volume of sample. The mixture was centrifuged and the supernatant kept for HPLC analysis. Amino acids were automatically derived with OrthoPhtalic Aldehyde (OPA) and 9-fluorenylmethyl-chloroformiate (FMOC-C1). The derivatives were separated on Hypersil AA-ODS column (Agilent Technologies) at 40°C by a linear gradient of acetate buffer (pH 7.2) with triethylamin (0.018%), tetrahydrofuran (0.3%) and acetonitrile. A diode array detector, at 338 nm for OPA derivatives and 262 nm for FMOC derivatives, was used.

### 1.3.Glutamate decarboxylase (GAD) activity:

50 mg of bacterial cells were harvested at 7 h of culture. Cells were washed twice with 0.2% KCl and suspended in sodium acetate buffer (100 mM, pH 4.6) containing 4.5 mM MgCl₂, 22% glycerol and 1.5 mM DTT. Cells were disrupted by sonication (four cycles of 30s and 60s spaced out, 6.5m/s) and kept on ice during the treatment. Cell debris were removed by centrifugation for 15 min at 10,000 *g* and 4°C.

The supernatant was used for enzyme assays, and the protein concentration of the extract was determined by the Bradford method with bovine serum albumin as the standard.

Enzyme assay was realized with 0.5 mL of substrate solution, consisting of 20 mM sodium glutamate, 2 mM pyridoxal phosphate (PLP) incubated at 30°C then mixed with 0.5 mL supernatant. Every 30 min until 4 h, 100 µL were sampled and inactivated by boiling for 5 min to stop the decarboxylation reaction. Reaction mixtures were subsequently analyzed for the presence of GABA using HPLC.

The GAD activity was measured at 7 h and neutral pH condition.

One unit of enzyme activity was defined here as the amount of enzyme which converted 1 µmole of glutamate per min and per mg of protein.

### 2. Results:

### 2.1.In vitro GABA production:

The biomass and GABA concentrations were measured all along the culture for NCDO2118, CNCM I-5388, CNCM I-5386 and NCDO2727 strains (Figure 2).

In the first 8 hours, the bacterium NCDO2118 as well as CNCM I-5388, CNCM I-5386 grew fast (growth rate around 0.7-0.8h⁻¹) at pH 6.6 compared to NCDO2727. GABA was accumulated at low levels for all strains. Then at 8 hours, the pH was lowered and controlled to 4.6, growth stopped thereafter and biomass decreased. This loss in cell viability was confirmed by plating.

Despite the growth arrest, the production of GABA by NCDO2118 strain or CNCM I-5388 and CNCM I-5386 continued with increased GABA production rate (Table 2). The maximal GABA concentration reached 60 mM at 24 hours for CNCM I-5388. Similar results were obtained with a second culture in bioreactor (independent duplicate). GABA production of 40-60 mM corresponds to the complete bioconversion of initial concentration of free glutamate into the bioreactor.

Despite the strong similarity of the *gad* operon sequence and organization in all the four strains, GABA production pattern of the NCDO2727 strain characterized under similar culture conditions was very different with a very weak amount of GABA produced since GABA concentration is always below 0.2 mM (Figure 2A) and GABA production rate is low (Table 2).

**Table 2: Specific rates of GABA production calculated between 7 and 11.5 hours (mmole/g cell dry weight/h) during growth of L. lactis NCDO2118, CNCM I-5388, CNCM I-5386 and NCDO2727 in bioreactor (in duplicates).**

| | NCDO2118 | NCDO2727 | CNCM I-5388 | CNCM I-5386 |
|---|---|---|---|---|
| µmax (h⁻¹) | 0.71 ± 0.05 | 0.21 ± 0.04 | 0.81 ± 0.08 | 0.71 ± 0.05 |
| ν ₘₑₐₙ GABA (mmole/g/h) | 2.86 ± 0.39 | 0.02 ± 0.04 | 6.70 ± 2.67 | 1.09 ± 0.27 |

### 2.2.In vitro GAD activity:

High GAD activity was obtained in the NCDO2118 strain consistently with its high GABA production ability, while in the NCDO2727 strain, the amount was extremely low (Table 3). Intermediary level of GAD activity was obtained for CNCM I-5386 strain while a very high activity was measured for the CNCM I-5388 strain. One can notice however that, under neutral pH condition at 7 hours, the GAD activity in the NCDO2118 strain, CNCM I-5388 or CNCM I-5386 was high despite the low GABA production accumulated in the bioreactor, suggesting that the enzymatic equipment for GABA production was already expressed in cells but not yet active.

**Table 3: Specific GAD activity (µmole/min.mg of protein) of the L. lactis strains after 7 hours of growth in M17 supplemented with free glutamate (8 g/L), arginine (5 g/L), and NaCl (300 mM) (n=3 for each of the two cultures replicates in bioreactor).**

| | NCDO2118 | NCDO2727 | CNCM I-5388 | CNCM I-5386 |
|---|---|---|---|---|
| GAD 7 hours | 42.7 ± 5.9 | 0.6 ±0.4 | 826.2 ±93.0 | 20.7 ±4.3 |

### EXAMPLE 2: Effect of L. lactis strains of interest in a stress model in rat (model IBS)

### 1. Materials and Methods:

### 1.1. Animals and surgical procedure:

Adult female Wistar rats (200-225 g) were purchased from Janvier Labs (Le Genest St Isle, France) and individually housed in polypropylene cages under standard conditions (temperature 22 ± 2°C and a 12-h light/dark cycle). Animals were allowed free access to water and food (standard pellets 2016, Envigo RMS SARL, Gannat, France). All experiments were approved by the Local Animal Care and Use Committee, in compliance with European directive 2010/63/UE.

Under general anesthesia by intraperitoneal administration of 0.6 mg/kg acepromazine (calmivet, Vetoquinol, Lure, France) and 120 mg/kg ketamine (Imalgene 1000, Merial, Lyon, France), animals were prepared for abdominal striated muscle electromyography (EMG) according to a previously described technique (Morteau *et al.,* 1994).

Three pairs of NiCr wire electrodes (60 cm length and 80 µm diameter) were implanted bilaterally in the striated muscles at 2 cm laterally from the midline. The free ends of electrodes were exteriorised on the back of the neck and protected by a glass tube attached to the skin.

### 1.2. Colorectal distension procedure and EMG recordings:

Female rats were accustomed to being in polypropylene tunnels (20 cm length and 7 cm diameter), where they could not move, escape or turn around, for several days before colorectal distension (CRD) in order to achieve familiarization with that environment.

A 4-cm long latex balloon, fixed on rigid catheter taken from an embolectomy probe (Fogarty), was used.

CRD which is a mechanical stimulus was performed after insertion of the balloon in the rectum at 1 cm from the anus. The tube was fixed at the basis of the tail. Isobaric distensions of the colon were performed from 0 to 60 mmHg by connecting the balloon to Distender Series IIR Barostat (G&J Electronics Inc, Toronto, Canada).

The first distension was performed at a pressure of 15 mmHg and an increment of 15 mmHg was added at each following step, until a maximal pressure of 60 mmHg was reached, each distension step lasting 5 min.

The striated muscle spike bursts, related to abdominal cramps, were recorded on an electroencephalograph machine (Mini VIII, Alvar, Paris, France) from implanted electrodes, using a short time constant (0.03 s) to remove low-frequency signals (<3 Hz). EMG recordings started 7 days after surgery.

The number of contractions for a period of 5 minutes represents the intensity of visceral pain.

### 1.3. Stress procedure:

All stress sessions were performed at the same time of day (between 10 am and 12 noon) to minimise any influence of circadian rhythm.

Partial restraint stress (PRS), a relatively mild non-ulcerogenic model of acute stress, was performed as previously described (Williams *et al.,* 1988).

Female rats were sedated with diethyl-ether and their fore shoulders, upper forelimbs and thoracic trunk were wrapped in a confining harness of paper tape to restrict, but not prevent, body movements.

Animals were then placed in their home cage for 2 h.

### 1.4. Experimental protocol:

Four series of experiments, based on a 10-day treatment by oral gavage, were conducted using, for each series, three groups of 7 to 12 female rats equipped for EMG.

Rats were given *L. lactis* NCDO2118 or NCDO2727 by gavage once daily for 10 days. Washed bacterial cells (10⁹ cfu per day) were used in order to minimize the amount of GABA initially administered to rats. Unless otherwise stated, free glutamate (0.2% (w/v)) was added to the gavage mixture to favor *in vivo* GABA production.

For all conditions, basal abdominal response to CRD was recorded on Day 9 of the treatment and PRS-induced visceral hypersensitivity to CRD recorded on Day 10 of the treatment.

CRD measurements were performed 20 minutes after the 2 h PRS session.

On vehicle-treated animals, free glutamate (0.2% (m/v)) is verified as having no effect *per se* on basal and stress-induced colorectal sensitivity.

### 1.5.Statistical methods:

For animal experiments, data are reported as the means ± standard errors of the means (SEM). The software GraphPad Prism 6.0 (GraphPad, San Diego, CA) was used for statistical analysis. One-way ANOVA, followed by Tukey's Multiple Comparison test, was performed to compare data between the different groups of animals. Statistical significance was accepted at **P** < 0.05.

### 2. Results:

### 2.1. Effect of free glutamate on basal and stress-induced colorectal sensitivity

In rats treated with the vehicle under basal conditions (*i.e.* before PRS), gradual colorectal distension (CRD) increased the frequency of abdominal contractions in a pressuredependent manner (Figure 3).

Compared with vehicle, administration of free glutamate did not affect the abdominal response to CRD (Figure 3).

In vehicle-treated rats, a 2-h PRS significantly increased the number of abdominal contractions in comparison with basal conditions; administration of free glutamate did not modify the visceral hypersensitivity response to CRD measured after the PRS session (Figure 3).

Thus, free glutamate had no effect *per se* on basal and stress-induced colorectal sensitivity.

### 2.2. Effect of the L. lactis NCDO2118 on visceral hypersensitivity

The influence of *L. lactis* NCDO2118 and NCDO2727 on stress-induced visceral hypersensitivity to CRD is shown in Figure 4.

In vehicle-treated rats, a 2-h PRS significantly increased the number of abdominal contractions compared to basal conditions for all the pressures of distention applied from 30 mmHg (P<0.001, Figure 4).

Importantly, a 10-day oral administration of the *L. lactis* NCDO2118 suppressed the PRS-induced enhancement of abdominal contractions (P<0.01 for a distension pressure of 60 mmHg, Figure 4), restoring a quasi-basal sensitivity to CRD.

In contrast, oral administration of the *L. lactis* NCDO2727 had no effect on stress-induced visceral hypersensitivity (Figure 4).

In conclusion, acute restraint stress induced colonic hypersensitivity to distension in rats and this hypersensitivity phenotype was reversed by *L. lactis* NCDO2118 but not by *L. lactis* NCDO2727 after a 10-day daily oral administration (10⁹ cfu per day).

### EXAMPLE 3: The prevention of the stress-induced visceral hypersensitivity by L. lactis NCDO2118 is due to its ability to deliver GABA in vivo (for reference purposes)

### 1. Materials and Methods:

### 1.1. Bacterial strains

The strains NCDO2118 and NCDO2727 were used.

*L. lactis* NCDO2118 Δ*gadB* was constructed by double crossing over in the chromosome.

The GAD encoding gene *gadB* was deleted in order to interrupt the GABA pathway.

Two fragments of 829 and 981 bp were PCR amplified just upstream and downstream of the *gadB* coding sequence, respectively, with NCDO2118 chromosome as a matrix. Primers are listed in Table 4. The two fragments were fused by overlapping PCR. The pGhost9 vector was PCR amplified and linked to the fused fragments using Gibson assembly method (New Englang Biolabs). Resulting plasmid was verified by sequencing and introduced in *L. lactis* NCDO2118. Chromosomal deletion of *gadB* was then obtained by double crossing over as previously described (Maguin *et al.,* 1996). Deletion of *gadB* sequence into the chromosome was verified by PCR.

The culture conditions of the strains are the same as the one described in Example 1.

**Table 4: Primers used for the inactivation of gadB in L. lactis NCDO2118.**

| Primer name | *5'-3' sequence* | SEQ ID NO: | Use for: |
|---|---|---|---|
| 821-GBgadCR | GGAATTCGATTTAGATGC CATAGGAGGATTTTC | 1 | Amplification of *gabB* upstream sequence |
| 894-GBgadC2F | GATGAATATCGTACATCC TCCAATTTTTTAATG | 2 | |
| 892-GBkefA2F | AAGCTTGATAAAACAAGA AAATATTCATGAAATTCA G | 3 | Amplification of *gabB* downstream sequence |
| 893-GBkefA2R | GGAGGATGTACGATATTC ATCTTAAGAAAAATCAAA AGC | 4 | |
| 822-GBpGhost9EVF | CATCTAAATCGAATTCCT GCAGCCCG | 5 | Amplification of pGhost9 backbone |
| 891-GBpGh9EV2R | TCTTGTTTTATCAAGCTTA TCGATACCGTC | 6 | |
| 898-amt GadB(DCO) | TTGGATTAGCTGCGGCAT ATTTTATCG | 7 | Verification of *gabB* deletion onto the chromosome |
| 899-avl GadB(DCO) | CCTTGTTGACCATAATGC AAAGCAGGT | 8 | |

### 1.2. Sequence analysis of gad operon:

In the NCDO2118 strain, the *gad* operon sequence was extracted from the chromosome sequence deposited in NCBI-GenBank database under the accession number CP009054.

To amplify the *gad* operon in NCDO2727 strain, two primers were used, GadSeq_F (5'-TCCAGAAATAACAGCTACATTGACATAATG-3') and GadSeq_R (5'-TAACAGCCCCATTATCTAAGATTACTCC-3'). The amplification was carried out with 20 ng of genomic DNA in 25-µL reaction mixture using the Q5 High-Fidelity DNA polymerase (NEB) and according to the manufacturer's recommendation. PCR conditions consisted of an initial denaturation step of 3 min at 98°C, followed by 30 cycles of 98°C for 10 sec, 64°C for 30 sec, and 72°C for 3 min and a final elongation step of 2 min at 72°C.

Then, the amplicon was purified using the QIAquick gel extraction kit (QIAgen).

The sequence was performed by Eurofins Genomics with a set of primers listed in Table 5. The sequence of the operon has been deposited in NCBI-GenBank database under the accession number MK225577.

Sequences of *gad* operon were compared by Blast alignment algorithm.

**Table 5: Primers used for the sequencing of gad operon in L. lactis NCDO2727.**

| Primer name | *5'-3' sequence* | SEQ ID NO: |
|---|---|---|
| GabSeq_2_F | AAAATATAGAAGGAGACTATTGCAAATA GC | 9 |
| GabSeq_2_R | AAAAATTAATGGCCATCGTTGGTAGTTCT C | 10 |
| GabSeq_3_F | TCTGTGCAGCAGAAATGGCGACGGTTGA A | 11 |
| GabSeq_3_R | TCCCCATAAATTTTTCTTTTTCACTCGCAT | 12 |
| GabSeq_4_F | CGGTTATTCCTCAAAAAGACTTATCATTA A | 13 |

### 2. Results:

With this deletion, and under the same culture conditions, the mutant could grow similarly to the wild type (growth rate of 0.65 h⁻¹). Under these conditions however, no GABA was produced (<0.29 mM at 24 h) and no GAD activity detected at 7 h.

The beneficial effect of *L. lactis* NCDO2118 treatment on stress-induced visceral hypersensitivity (*P*<0.001) was reversed when animals received the NCDO2118 *ΔgadB* isogenic strain (Figure 6, *P*<0.05 NCDO2118 *ΔgadB- vs.* NCDO2118-treated animals).

### EXAMPLE 4: Comparison of the effects obtained on visceral hypersensitivity with L. lactis strains of interest in a stress model in rat (model IBS)

### 1. Materials and Methods:

The preparation of the animals is the same as the one described in Example 2.

Rats were given *L. lactis* NCDO2118 or CNCM I-5388 by gavage once daily for 4 or 9 days. Washed bacterial cells (10⁹ cfu per day) were used in order to minimize the amount of GABA initially administered to rats. Unless otherwise stated, free glutamate (0.2% (w/v)) was added to the gavage mixture to favor *in vivo* GABA production.

### 2. Results:

The influence over time of *L. lactis* NCDO2118, CNCM I-5388 and CNCM I-5388 *Δgad* on stress-induced visceral hypersensitivity to CRD is shown in Figures 5 and 7.

CNCM I-5388 and NCDO2118 in the presence of glutamate reduce the visceral hypersensitivity to the stress.

### REFERENCES

- Ait-Belgnaoui A. et al., Pain. 2005;113:141-7.
- Bouin M. et al., Dig. Dis. Sci. 2001; 46:2542-2548.
- Camilleri M. et al., Clin. Gastroenterol. Hepatol. 2008; 6:772-781.
- Camilleri M et al., Am J Physiol Gastrointest Liver Physiol. 2012;303:G775-85.
- Chang L. et al., Gastroenterology. 2011; 140:761-765.
- Coculescu E. et al., J. Med. Life. 2014; 7:512-515.
- Craig O. et al., Curr Opin Gastroenterol. 2018; 34:50-56.
- Dean B.B. et al., Am J Manag Care. 2005; 11:S17-26.
- de Castro LA. et al.,Pain Med. 2014; 15: 2164-5.
- Didari T. et al., World. J. Gastroenterol. 2015; 21:3072-3084.
- Ford A.C. et al., Am. J. Gastroenterol. 2014; 109:1547-1561.
- Gué M. et al., Neurogastroenterol Motil. 1997;9(4):271-9.
- Hungin A.P. et al., Aliment. Pharmacol. Ther. 2013; 38:864-886.
- Keszthelyi D. et al., EJP. 2012; 16: 1444-1454.
- Kuiken S.D. et al., Aliment. Pharmacol. Ther. 2005; 22:157-164.
- Laroute V. et al., Microorganisms. 2017; 5.
- Maguin E. et al., J. Bacteriol. 1996; 178:931-935.
- Mayer E.A. et al., Gastroenterology. 1994; 107:271-293.
- Mayer E.A. et al., Gut. 2008; 57:384-404.
- Mazurak N. et al., J. Neurogastroenterol. Motil. 2015; 21:471-485.
- Mills S. et al., Int. J. Dairy Technol. 2010; 63:149-170.
- Miziara ID. et al., J. Psychosom. Res., 2009; 62:443-448.
- Moayyedi P. et al., Gut. 2010; 59:325-332.
- Morteau O. et al., Dig. Dis. Sci. 1994; 39:1239-1248.
- Nomura M. et al., Int. J. Syst. Bacteriol. 1999; 49:163-166.
- Pérez-Berezo T. et al., Nat. Commun. 2017; 8, 1314.
- Pokusaeva K. et al., Neurogastroenterol. Motil. 2017; 29, e12904.
- Posserud I. et al., Gastroenterology. 2007; 133:1113-1123.
- Radziwill-Bienkowska et al., Appl. Microbiol. Biotechnol. 2016; 100:9605-9617.
- Sun Y. et al., Chin J Dig Dis. 2006;7:149-55.
- Sun A. et al., J. Oral Pathol. Med., 2013; 42:649-655.
- Wang Y. et al., J. Microbiol. Methods. 2011; 86:390-392.
- Williams C.L. et al., Am J Physiol. 1987; 253:G582-6.
- Williams C.L. et al., Gastroenterology. 1988; 94:611-621.
- Zhang C. et al., ISME J. 2016; 10:2235-2245.
- Zhou Q. et al., Pain. 2010; 148:454-461.

## Claims

1. A *Lactococcus lactis* strain selected in the group consisting of: CNCM I-5388, CNCM I-5386 for use in the prevention and/or the treatment of abdominal or visceral pain.

2. The *Lactococcus lactis* strain for use according to claim 1, **characterized in that** said strain is administered in presence of free glutamate.

3. A cell fraction obtained from the *Lactococcus lactis* strain as defined in claim 1, for use in the prevention and/or the treatment of visceral pain.

4. A composition comprising the *Lactococcus lactis* strain as defined in claim 1 or the cell fraction as defined in claim 3, for use in the prevention and/or the treatment of visceral pain.

5. The composition for use comprising the *Lactococcus lactis* strain according to claim 4, **characterized in that** it comprises a content of the *L. lactis* strain allowing the administration of at least 10⁶ colony forming units (cfu) of said *L*. *lactis* strain per day.

6. The composition for use according to claim 4 or claim 5, **characterized in that** it also comprises free glutamate.

7. The composition for use according to anyone of claims 4 to 6, **characterized in that** it is a food product.

8. The *Lactococcus lactis* strain, cell fraction, or composition, for use according to claims 1, 3 or 4-7 respectively, **characterized in that** visceral pain is linked to a psychological stress events and/or anxiety.

9. The *Lactococcus lactis* strain according to claim 1 or the cell fraction according to claim 3 or the composition according to any one of claim 4 to 7 for their use according to claim 8, **characterized in that** visceral pain is due to burning mouth syndrome (BMS).

10. A *Lactococcus lactis* strain, **characterized in that** said strain is selected in the group consisting of: CNCM I-5388, CNCM I-5386.

11. A cell fraction obtained from the *Lactococcus lactis* strain as defined in claim 10.

12. A composition comprising the *Lactococcus lactis* strain according to claim 10 or the cell fraction according to claim 11.

13. The composition comprising the *Lactococcus lactis* strain according to claim 12, **characterized in that** it comprises at least 10⁶ cfu per gram dry weight.

14. The composition according to claim 12 or 13, **characterized in that** it is a food product.

15. The composition according to claim 12 or 13 for use as a medicament.

## Patentansprüche

1. *Lactococcus lactis-Stamm,* ausgewählt aus der Gruppe bestehend aus: CNCM I-5388, CNCM 1-5386 zur Verwendung in der Prävention und/oder Behandlung von Bauch- oder viszeralen Schmerzen.

2. *Lactococcus* lactis-Stamm zur Verwendung nach Anspruch 1, **dadurch gekennzeichnet, dass** der Stamm in Gegenwart von freiem Glutamat verabreicht wird.

3. Zellfraktion, die aus dem nach Anspruch 1 definierten *Lactococcus lactis*-Stamm erhalten wird, zur Verwendung in der Prävention und/oder Behandlung von viszeralen Schmerzen.

4. Zusammensetzung, die den *Lactococcus* lactis-Stamm nach Anspruch 1 oder die Zellfraktion nach Anspruch 3 umfasst, zur Verwendung bei der Prävention und/oder Behandlung von viszeralen Schmerzen.

5. Zusammensetzung zur Verwendung, die den *Lactococcus lactis* -Stamm nach Anspruch 4 umfasst, **dadurch gekennzeichnet, dass** sie einen Gehalt an dem *L. lactis -* Stamm umfasst, der die Verabreichung von mindestens 10⁶ koloniebildenden Einheiten (cfu) des *L. lactis*-Stammes pro Tag ermöglicht.

6. Zusammensetzung zur Verwendung nach Anspruch 4 oder 5, **dadurch gekennzeichnet, dass** sie auch freies Glutamat umfasst.

7. Zusammensetzung zur Verwendung nach einem der Ansprüche 4 bis 6, **dadurch gekennzeichnet, dass** es sich um ein Lebensmittel handelt.

8. *Lactococcus* lactis-Stamm, Zellfraktion oder Zusammensetzung, zur Verwendung jeweils nach den Ansprüchen 1, 3 oder 4-7, **dadurch gekennzeichnet, dass** viszerale Schmerzen mit psychischen Stressereignissen und/oder Angst verknüpft sind.

9. *Lactococcus lactis-Stamm* nach Anspruch 1 oder Zellfraktion nach Anspruch 3 oder Zusammensetzung nach einem der Ansprüche 4 bis 7 zur Verwendung derselben nach Anspruch 8, **dadurch gekennzeichnet, dass** der viszerale Schmerz auf das Burning-Mouth-Syndrom (BMS) zurückzuführen ist.

10. *Lactococcus* lactis-Stamm, **dadurch gekennzeichnet, dass** der Stamm aus der Gruppe ausgewählt wird, bestehend aus: CNCM 1-5388, CNCM 1-5386.

11. Zellfraktion, die aus dem nach Anspruch 10 definierten *Lactococcus lactis*-Stamm erhalten wurde.

12. Zusammensetzung, die den *Lactococcus* lactis-Stamm nach Anspruch 10 oder die Zellfraktion nach Anspruch 11 umfasst.

13. Zusammensetzung, die den *Lactococcus* lactis-Stamm nach Anspruch 12 umfasst, **dadurch gekennzeichnet, dass** sie mindestens 10⁶ cfu pro Gramm Trockengewicht umfasst.

14. Zusammensetzung nach Anspruch 12 oder 13, **dadurch gekennzeichnet, dass** es sich um ein Lebensmittel handelt.

15. Zusammensetzung nach Anspruch 12 oder 13 zur Verwendung als Arzneimittel.

## Revendications

1. Souche de *Lactococcus lactis* sélectionnée dans le groupe constitué de : CNCM 1-5388, CNCM 1-5386, destinée à être utilisée dans la prévention et/ou le traitement de la douleur abdominale ou viscérale.

2. Souche de *Lactococcus lactis* destinée à être utilisée selon la revendication 1, **caractérisée en ce que** ladite souche est administrée en présence de glutamate libre.

3. Fraction cellulaire obtenue à partir de la souche de *Lactococcus lactis* telle que définie dans la revendication 1, destinée à être utilisée dans la prévention et/ou le traitement de la douleur viscérale.

4. Composition comprenant la souche de *Lactococcus lactis* telle que définie dans la revendication 1 ou la fraction cellulaire telle que définie dans la revendication 3, destinée à être utilisée dans la prévention et/ou le traitement de la douleur viscérale.

5. Composition destinée à être utilisée comprenant la souche de *Lactococcus lactis* selon la revendication 4, **caractérisée en ce qu'**elle comprend une teneur en souche de L. *lactis* permettant l'administration d'au moins 10⁶ unités formant des colonies (ufc) de ladite souche de *L. lactis* par jour.

6. Composition destinée à être utilisée selon la revendication 4 ou la revendication 5, **caractérisée en ce qu'**elle comprend également du glutamate libre.

7. Composition destinée à être utilisée selon l'une quelconque des revendications 4 à 6, **caractérisée en ce qu'**il s'agit d'un produit alimentaire.

8. Souche de *Lactococcus lactis,* fraction cellulaire ou composition, destinée à être utilisée selon les revendications 1, 3 ou 4 à 7 respectivement, **caractérisée en ce que** la douleur viscérale est liée à des événements de stress psychologique et/ou d'anxiété.

9. Souche de *Lactococcus lactis* selon la revendication 1 ou la fraction cellulaire selon la revendication 3 ou la composition selon l'une quelconque des revendications 4 à 7 pour leur utilisation selon la revendication 8, **caractérisée en ce que** la douleur viscérale est due au syndrome de la bouche brûlante (BMS).

10. Souche de *Lactococcus lactis,* **caractérisée en ce que** ladite souche est choisie dans le groupe constitué de : CNCM 1-5388, CNCM 1-5386.

11. Fraction cellulaire obtenue à partir de la souche de *Lactococcus lactis* telle que définie dans la revendication 10.

12. Composition comprenant la souche de *Lactococcus lactis* selon la revendication 10 ou la fraction cellulaire selon la revendication 11.

13. Composition comprenant la souche de *Lactococcus lactis* selon la revendication 12, **caractérisée en ce qu'**elle comprend au moins 10⁶ ufc par gramme de poids sec.

14. Composition selon la revendication 12 ou 13, **caractérisée en ce qu'**il s'agit d'un produit alimentaire.

15. Composition selon la revendication 12 ou 13 destinée à être utilisée comme médicament.
